# EUROPEAN PATENT APPLICATION

(11) **EP 1 283 262 A1**
(43) Date of publication of application: **12.02.2003**
(21) Application number: 01917711.2
(22) Date of filing: 30.03.2001
(51) Int. Cl.: C12N 15/12, C12N 15/67, C07K 14/48, C12Q 1/02

(54) **NEUROTROPHIC FACTOR EXPRESSION-INDUCING AGENT**

(30) Priority: 31.03.2000 JP 2000101300
(71) Applicant: Helix Research Institute, Kisarazu-shi, Chiba 292-0812 (JP); Mitsubishi Pharma Corporation, Osaka-shi, Osaka 541-0046 (JP)
(72) Inventor: MITSUMORI, Cristina, Sodegaura-shi, Chiba 299-0257 (JP); MORIKAWA, Noriyuki, Kisarazu-shi, Chiba 292-0833 (JP); HAYASHI, Koji, Toyonaka-shi, Osaka 561-0802 (JP); NAGAHARI, Kenji, Suginami-ku, Tokyo 174-0000 (JP); OTA, Toshio, Fujisawa-shi, Kanagawa 251-0042 (JP); HIO, Yuri, Kisarazu-shi, Chiba 292-0812 (JP); NISHIKAWA, Tetsuo, Itabashi-ku, Tokyo 173-0013 (JP); ISOGAI, Takao, Inashiki-gun, Ibaraki 300-0303 (JP); KAWASAKI, Masakazu, c/o Mitsubishi Pharma Corp., Chuo-ku,Tokyo 103-8405 (JP); HASHIMOTO, Kenji, c/o Mitsubishi Pharma Corpor., Chuo-ku,Tokyo 103-8405 (JP); KISHIMOTO, Toshimitsu, c/o Mitsubishi Pharma Corp., Chuo-ku, Tokyo 103-8405 (JP)
(74) Representative: Bohmann, Armin K., Dr.
(86) International application number: JP0102768
(87) International publication number: WO01073024

(57) **Abstract**

The culture supernatant of COS cells expressing the PSEC56 gene was found to have a neurite extension effect. The main substance of the activity contained in this culture supernatant was revealed to be NGF. It was shown that PSEC56 can be utilized as a neurotrophic factor expression-inducing agent, and that the NGF induction system can be utilized for drug development against diseases of the nervous system.

## Description

### Technical Field

The present invention relates to neurotrophic factor expression-inducing agents, a method for inducing the expression of neurotrophic factors, and a method of screening for compounds that regulate the induction of neurotrophic factor expression.

### Background Art

Neurotrophic factor (NTF) is a factor that has the activity to maintain the survival and function of neurons, and promoting neurites extension. Currently, great attention has been paid on the factor as a target for drug development of nerve related diseases.

So far, neurotrophic factors, such as nerve growth factor (NGF), brain-derived neurotrophic factor (BDNF), NT-3, and NT-4/5, have been isolated. NGF, BDNF, NT-3, and NT-4/5 share a very similar physical and chemical structure with a similar physiological function, and are generically called neurotrophins. In addition, various cytokines, such as ciliary neurotrophic factor (CNTF), basic fibroblast growth factor (bFGF), epidermal growth factor (EGF), glial cell line-derived neurotrophic factor (GDNF), insulin-like growth factor-I and -II (IGF-I and -II, respectively) , and interleukin-6 (IL-6) have been isolated.

Generally, administration of these isolated neurotrophic factors is widely tried to treat diseases accompanying neuronal degeneration and neuronal cell death, regardless of whether they belong to the central or peripheral nervous system. Furthermore, recently, although it is in the preclinical stages, gene therapy by neurotrophic factors is being planned. Moreover, use of neurotrophic factors for elevating the adhesion rate during transplantation of fetal neuron to the brain is also considered.

For example, the use of nerve growth factor (NGF) (Genentech/Hoffmann-La Roche) on diabetic peripheral neuropathy in clinical development reached phase III but did not proceed any further since no significant difference could be obtained. Currently, clinical application of NGF on HIV encephalopathy is proceeding. Further, no clinical effectiveness of brain-derived neurotrophic factor (BDNF) (Regeneron/Amgen, Sumitomo, Medtronic) on amyotrophic lateral sclerosis could be confirmed. However, further clinical tests are planned for BDNF on other general neuropathies, especially diabetic peripheral neuropathy. Regarding NT-3 (Regeneron/Amgen), clinical tests targeting diabetic peripheral neuropathy, amyotrophic lateral sclerosis, and such are in progress. Furthermore, clinical tests of ciliary neurotrophic factor (CNTF) (Cyto Therapeutics) on amyotrophic lateral sclerosis and Huntington's disease are in progress.

On the other hand, drug development has been attempted using not only neurotrophic factors themselves, but also factors that induce the expression of a neurotrophic factor. At present, the production mechanism of neurotrophic factors and several compounds that promote the production of the factors are known. For example, a β2,3 agonist, clenbuterol, is known to promote NGF expression in glial cells via a β-adrenaline receptor. Increase of cyclic-adenosine monophosphate (cAMP) followed by the activation of cAMP-activated protein kinase (PKA) is reported to be involved in the pathway after adrenaline receptor stimulation. However, details of the pathway that ultimately leads to the expression of NGF are unknown. The activation of PKA via the β-adrenaline receptor is only an example of the pathways of NGF expression, and other molecular mechanisms involving various chemical substance and test systems that regulate the expression of neurotrophic factors, such as NGF, have been reported. Such mechanisms include pathways such as those involving PKA activation and cAMP increase by forskolin; those involving calcium-dependent protein kinase (PKC) activation by diacylglycerol and phorbol ester; and those via sphingomyelin involving vitamin D3, interleukin-1β (IL-1β), lipopolysaccharide, and ceramide (Semkova, I. and Krieglstein, J. (1999) Brain Res. Rev. 30, 176-188). In addition, aFGF is known to induce NGF production from glial cells (Neuroscience Letters 126, 18-20 (1991)). Furthermore, IL-1 and catecholamine are also known to induce the expression of NGF.

Xaliproden (SR-57746) is an example of neurotrophic factor expression-inducing agent that is being developed as a medicament. This substance is basically a 5-HT1A receptor agonist, and is reported to enhance NGF production via an unknown action mechanism to improve neuropathy in animal models. Clinical tests of Xaliproden for amyotropic lateral sclerosis and Alzheimer's disease are in progress. Application thereof to gene therapy by neurotrophic factors are also being considered.

As described above, drug development is being carried out regarding neurotrophic factors themselves and agents inducing them. Considering diseases of the central nervous system, however, neurotrophic factors are proteins of high molecular weight, and thus cannot pass through the blood brain barrier (BBB) in their original form. Therefore, when the neutrophic factors are administered to the periphery, they do not translocate into the brain and no effect against diseases of the central nervous system can be expected. Thus, the neurotrophic factors have to be injected directly into the brain for administration. On the other hand, inducing agents, if they are small molecules that go through the blood brain barrier, are expected to show effects on diseases of the central nervous system. Moreover, almost no effect can be anticipated by the administration of neurotrophic factors against diseases of the peripheral nervous system according to the results of clinical tests on peripheral neuropathy. Therefore, enhancing production of neurotrophic factors using inducing agents is more promising than the use of neurotrophic factors themselves.

Furthermore, continuous administration to the affected area is required for direct administration of neurotrophic factors. In contrast, constitutive expression of neurotrophic factors can be expected gene therapy and/or administration of inducing agents. Furthermore, if the inducing agents are small molecules, simplified (oral, subcutaneous, and such) administration enables maintenance of a necessary concentration *in vivo*, and as a consequence, constitutive expression of the neurotrophic factors can be expected.

Hitherto, regardless of their utility as medicaments, reports on neurotrophic factor expression-inducing agents are still few, and development of novel neuotrophic factor expression-inducing agents are desired.

### Disclosure of the Invention

This present invention has been made in view of such situation, and its objective is to identify factors that induce the expression of neurotrophic factors to provide neurotrophic factor expression-inducing agents, method for inducing the expression of neurotrophic factors, and method of screening for compounds that regulate the induction of neurotrophic factor expression that utilize these identified factors.

The present inventors treated NT-2 nerve progenitor cells, human fetal testis-derived teratocarcinoma cells, with retinoic acid, prepared mRNAs from the treated cells, and then cloned a plurality of full-length cDNAs from the mRNAs by the oligo-capping method. The nucleotide sequences of these full-length cDNAs were analyzed, and a novel gene (PSEC56) with a secretory signal was discovered among them.

The inventors transfected the gene into COS cells, and examined the activity of the culture supernatant of COS cells expressing the gene on dorsal root ganglion (DRG) cells . As a result, this culture supernatant was revealed to have a neurite extension effect towards these cells. On the other hand, PSEC56 protein itself purified by metal affinity column from the culture supernatant did not show any neurite extension effect on DRG cells.

Accordingly, the present inventors performed analyses to identify the main substance of the activity included in this culture supernatant. As a result, the neurite extension effect of the culture supernatant on DRG cells was suppressed by treating the culture supernatant of PSEC56 transfected COS-7 cells with antibodies against NGF. Moreover, NGF was detected in the culture supernatant of COS-7 cells transfected with the PSEC56 gene. That is, the present inventors demonstrated NGF as the main substance of activity within the culture supernatant.

As described above, since NGF expression was induced by intracellular expression of PSEC56 protein, the PSEC56 protein and genes encoding said protein can be utilized as inducing agents of NGF expression in cells. Furthermore, the inducing system of NGF expression by PSEC56 protein expression may be also utilized for the development of therapeutic agents and preventive agents for diseases of the nervous system.

This invention is based on the above findings and specifically provides:
(1) a neurotrophic factor expression-inducing agent, wherein PSEC56 protein or DNA encoding said protein serves as an active ingredient;
(2) the agent of (1), wherein the neurotrophic factor is NGF;
(3) a method for inducing the expression of neurotrophic factors, which comprises the step of expressing PSEC56 protein;
(4) the method of (3), wherein the neurotrophic factor is NGF;
(5) a method of screening for compounds that regulate the induction of neurotrophic factor expression by PSEC56 protein, which comprises the steps of:
   (a) contacting a test sample with a transfected cell including an expressive DNA encoding the PSEC56 protein;
   (b) detecting the expression of the neurotrophic factor in said cell; and
   (c) selecting the compound that enhances or suppresses the induction of neurotrophic factor expression compared to the expression detected in the absence of the test sample (control); and
(6) the method of (5), wherein the neurotrophic factor is NGF.

This invention provides neurotrophic factor expression-inducing agents, wherein PSEC56 protein or DNA encoding the protein serves as an active ingredient.

The active ingredient of an expression-inducing agent of this invention, "PSEC56 protein", includes the PSEC56 protein of SEQ ID NO: 2, as well as proteins functionally equivalent thereto. Herein, "proteins functionally equivalent to PSEC56 protein of SEQ ID NO: 2" refers to proteins that are highly homologous, at the primary structure, to the PSEC56 protein of SEQ ID NO: 2 at the primary structure, and which, similarly to the PSEC56 protein of SEQ ID NO: 2, have the activity to induce neurotrophic factor expression upon their expression. It may be any protein with any kind of name. Herein, the term "highly homologous" refers to a sequence identity of at least 40% or more, preferably 60% or more, and more preferably 80% or more (for example, 90% or more, or 95% or more). Examples of such proteins are mutants and variants of the PSEC56 protein of SEQ ID NO: 2, and homologous proteins derived from other organisms, but are not limited to these examples . The activity of a test protein to induce neurotrophic factors can be detected, for example, by expressing the test protein in COS cells, and detecting, as an indicator, secretion of neurotrophic factors, such as NGF, into the culture supernatant (see Example 2).

A protein that is functionally equivalent to the PSEC56 protein of SEQ ID NO: 2 can be prepared by one skilled in the art by, for example, a method for introducing mutations to the amino acid sequence of a protein (for example, site-directed mutagenesis (Current Protocols in Molecular Biology edit. Ausubel et al. (1987) Publish. John Wiley & Sons Section 8.1-8.5)). Such proteins may also be produced by naturally occurring amino acid mutations. The "PSEC56 protein" of this invention includes proteins that are functionally equivalent to the PSEC56 protein of SEQ ID NO: 2, in which one or more amino acids of the PSEC56 protein of SEQ ID NO: 2 have been substituted, deleted, inserted, and/or added. There are no limitations on the number of mutations and the sites of mutation of amino acids in the protein as long as its function is maintained. Typically, the number of mutations is 10% or less of all amino acids, preferably 5% or less of all amino acids, and more preferably 1% or less of all amino acids. Proteins with deletion of amino acids from the PSEC56 protein include, for example, proteins in which a signal peptide has been removed.

Additionally, a protein that is functionally equivalent to the PSEC56 protein of SEQ ID NO: 2 can be isolated using hybridization techniques or gene amplification techniques well known to those skilled in the art. That is, one skilled in the art can ordinarily isolate a DNA highly homologous to a probe from biological samples including human, rat, mouse, and such; and then, prepare the corresponding protein from the isolated DNA utilizing hybridization techniques wherein the DNA (SEQ ID NO: 1) encoding the PSEC56 protein of SEQ ID NO: 2 or parts thereof is used as a probe (Current Protocols in Molecular Biology edit. Ausubel et al. (1987) Publish. John Wiley & Sons Section 6.3-6.4).

A stringent hybridization condition for isolating DNAs encoding such proteins is usually "1x SSC, 0.1% SDS, 37°C" or so, a more stringent condition "0.5x SSC, 0.1% SDS, 42°C" or so, and an even more stringent condition "0.2x SSC, 0.1% SDS, 65°C" or so. The more stringent the condition for hybridization, the more efficient isolation of a DNA with a higher homology with the probe sequence is expected. However, the above-mentioned combinations of SSC, SDS, and temperature conditions are only examples, and one skilled in the art can accomplish similar stringencies to those mentioned above by appropriately combining the above-mentioned and other factors (for example, probe concentration, probe length, hybridization reaction time, and so on) that determine the stringency of hybridization.

Alternatively, gene amplification techniques (PCR) (Current Protocols in Molecular Biology edit. Ausubel et al. (1987) Publish. John Wiley & Sons Section 6.1-6.4) may be used to amplify and isolate DNAs that are highly homologous to the DNA sequence (SEQ ID NO: 1) encoding the PSEC56 protein of SEQ ID NO: 2 or parts thereof by designing primers based on the information of the DNA sequence encoding the PSEC56 protein. Furthermore, using the isolated DNA, proteins that are functionally equivalent to the PSEC56 protein of SEQ ID NO: 2 can be obtained.

Proteins encoded by the DNAs that are isolated using such hybridization techniques and gene amplification techniques usually have high sequence homology at the amino acid sequence level to the PSEC56 protein of SEQ ID NO: 2.

A nucleotide sequence is determined as "homologous" by performing a sequence homology search with BLAST N, for example, when the sequence shows a homology of 40% or higher to a region of 400 bp or longer of the 1746 bp DNA region encoding the PSEC56 protein. On the other hand, an amino acid sequence can be determined "homologous" by performing a sequence homology search with BLAST X, for example, when the sequence indicates a homology of 30% or higher to a region of 80 residues or longer of the 582 residue of the PSEC56 protein. Furthermore, sequence homologies can be determined using other search algorithms, such as FASTA and Smith-Waterman (GenBank (http://www.ncbi.nlm.nih.gov/web/GenBank/)).

At the nucleotide sequence level, the PSEC56 sequence (see SEQ ID NOs: 1 and 2) shows sequence homologies of 49%, 48%, and 48% at 185 bp- 751 bp, 552 bp- 986 bp, and 1213 bp- 1635 bp, respectively, to human FKBP12. The PSEC56 has a primary structure wherein three FKBP domains are aligned. In addition, on the C terminus of the amino acid sequence, a "His-Glu-Glu-Leu" sequence, which is predicted to be an endoplasmic reticulum translocation signal, has been confirmed. The PSEC56 sequence has 76% sequence homology (1-2632 bp), and 88% amino acid homology (1-582 residues) with mouse FKBP65.

As described above, the active ingredient of the expression-inducing agent of the present invention include proteins that are functionally equivalent to the PSEC56 protein of SEQ ID NO: 2, which proteins are encoded by DNAs that hybridize with a DNA encoding the PSEC56 protein of SEQ ID NO: 2.

The PSEC56 protein can be prepared as a recombinant protein, or as a naturally occurring protein. The recombinant protein can be prepared, for example, by introducing a vector to which a DNA encoding the PSEC56 protein has been inserted to an appropriate host cell, and then purifying the proteins expressed in the transfectant or those secreted into the culture supernatant. On the other hand, the naturally occurring protein can be prepared, for example, using an affinity column bound to antibodies against the PSEC56 protein (Current Protocols in Molecular Biology edit. Ausubel et al. (1987) Publish. John Wiley & Sons Section 16.1-16.19).

There are no particular limitations on the form of the "DNA encoding the PSEC56 protein", which is an active ingredient of the expression-inducing agent of this invention, and includes cDNAs, as well as genomic DNAs, chemically synthesized DNAs, etc. The DNAs of this invention can be prepared by standard methods, such as hybridization methods using a DNA sequence (for example, SEQ ID NO: 1) encoding the PSEC56 protein or parts thereof as a probe, or PCR methods using primers synthesized based on the DNA sequence information.

There are no particular limitations on the form of the "DNA encoding the PSEC56 protein", which is an active ingredient of the expression-inducing agent of this invention, so long as it is intracellularly expressible. Preferably, the DNA is inserted into a vector that ensures its intracellular expression. Preferably used vectors include pME18S FL3, pcDNA3.1(-), pcDNA3.1(+), etc.

The expression-inducing agent of this invention may be used as a reagent for experimental research. Alternatively, the agent can be used as a medicament for treatment or prevention of diseases, such as diseases of the nervous system. That is, the "expression-inducing agent" of this invention includes both reagents and medicaments.

Examples of neurotrophic factors to be induced by the expression-inducing agent of this invention are BDNF, NT-3, NT-4/5, CNTF, GDNF besides NGF, but are not limited to these examples. The term "neurotrophic factors" herein refer to cell growth factors that act on cerebral nervous system and especially those having growth, differentiation, and (survival and/or functional) maintenance effects on neurons.

The present invention also provides a method to induce the expression of neurotrophic factors. This method includes the step of expressing the PSEC56 protein. There are no particular limitations on cells that are applicable for this method, and various cells are used according to the objective. For example, glial cells and Schwann cells that produce neurotrophic factors may be mainly used; but the present invention is not limited to these examples. To express the PSEC56 protein in a cell, for example, a DNA encoding the PSEC56 protein is inserted into a vector that ensures intracellular expression, and the vector is transfected into a target cell. Examples of conventional methods for the transfection of a vector into a cell includes the lipofectamine method, the calcium phosphate precipitation method, the electroporation method, the microinjection method, etc., but are not limited to these examples. For administration of a vector to patients, vectors derived from viruses such as retrovirus, adenovirus, and Sendai virus; and non-viral vectors such as liposomes may be used. In such cases, besides in *vivo* administration, ex *vivo* administration may also be conducted.

Additionally, the present invention provides a method of screening for compounds that regulate the induction of neutrophic factor expression caused by the PSEC56 protein. The screening method of this invention is characterized by the steps of adding a test sample to the above-mentioned expression induction system of neurotrophic factors, and then evaluating the effect of the test sample on the neurotrophic factor expression in response to PSEC56 expression. More specifically, the screening method of this invention can be carried out by the steps of: (a) contacting a test sample with a transfected cell that includes an expressive DNA encoding the PSEC56 protein; (b) detecting neurotrophic factor expression in the cell; and (c) selecting the compound that enhances or suppresses the induction of neurotrophic factor expression compared to the expression detected in the absence of the test sample.

Transfected cells used for the screening can be prepared by transfecting a cell with a vector to which a DNA encoding the PSEC56 protein has been inserted by the above-mentioned well-known methods for gene transfection. There are no particular limitations on the host cell to be transfected with a vector. Various cells are used according to the objective, and for example, COS cells and CHO cells are preferably used as eukaryotic cells for enhanced expression of the protein of interest. Alternatively, *in vivo* cells are exemplified by glial cells and Schwann cells that produce neurotrophic factors, but are not limited thereto.

There are no particular limitations on the test samples that are contacted with the cells prepared in this manner. For example, cell extracts, expression products of gene libraries, synthetic low molecular weight compounds, synthetic polypeptides, and naturally occurring compounds may be used, but is not limited to these examples.

According to the screening method of the present invention, the expression of a neurotrophic factor is detected after the contact of cells with a test sample. The expression of neurotrophic factors can be detected by performing Western Blotting using antibodies against the neurotrophic factors on the culture supernatant of transfected cells with the PSEC56 gene.

As a result of the detection, if the expression level of a neurotrophic factor is elevated compared to the level detected in the absence of the contact with a test sample (control), the used test sample is determined to promote the induction of the expression of the neurotrophic factor; and on the contrary, if the expression level of the neurotrophic factor is diminished compared to the control, the used test sample is determined to suppress the induction of the neurotrophic factor expression.

The PSEC56 protein and genes encoding the protein, as well as compounds isolated by the screening method of the present invention are expected to be applicable as therapeutic drugs and/or preventive drugs for spinal injury and diseases of the peripheral nerves, as well as diseases of the central nerve system including dementia, cerebral infarction, etc.

When using the PSEC56 gene for gene therapy, virus vectors such as retrovirus vector and adenovirus vector, or non-virus vectors such as liposomes, are used for administration to patients. Such administration methods include *in vivo* methods and ex vivo methods.

Alternatively, when using the PSEC56 protein and compounds isolated by the screening method of the present invention as medicaments, besides directly administering them to patients, they can be administered upon formulation by conventional preparation methods. For example, they can be administered orally or parenterally in forms such as tablets, capsules, granules, injections, and drops that are obtained via a normal preparation of medicinal components by mixing with pharmaceutical acceptable carriers (fillers, binders, disintegrators, corrigents, flavors, emulsifiers, and such), diluents, solubilizers, etc.

Tablets for oral administration include normally used carriers, such as sucrose, lactose, mannitol, maltitol, dextran, corn starch, and such; and typically include lubricants, such as magnesium stearate; preservatives, such as parabens and sorbic acids; antioxidants, such as ascorbic acid, α-tocopherol, and cysteine; disintegrators; binders; etc. Lactose and dried corn starch are effective diluents for capsule for oral administration.

Ordinary parenteral administrations, such as intravenous injections, intraperitoneal injections, and infusions, are prepared by appropriately adjusting the pH of the active ingredient solution, and then buffering and sterilizing the solution. Examples of vehicles and solvents that may be used include distilled water, Ringer solution, isotonic saline solution, etc. For use as intravenous injection, the total concentration of solute should be adjusted to make the solution isotonic.

The dose for administration is determined by taking the age, weight, administration time, method of administration, combination of drugs, and the condition of the disease of a patient to be treated, and other factors into consideration. The daily dose differs depending on the condition and weight of the patient, type of compound, administration route, and such, but for example, approximately 0.01 mg/patient/day to 1000 mg/patient/day, preferably 0.1 mg/patient/day to 300 mg/patient/day is preferred for oral administration; and approximately a dose of 0.01 mg/patient/day to 50 mg/patient/day, preferably 0.01 mg/patient/day to 10 mg/patient/day is preferably administered subcutaneously or intravenously for parenteral administration.

### Brief Description of the Drawings

Fig. 1 shows the structure of the pME18SFL3 cloning vector. The stuffer was excised with DraIII and the cDNA was inserted into that region.
Fig. 2 shows the neurite extension effect of the culture supernatant of COS-7 cells transfected with PSEC56, measured by cultivating DRG cells in a media containing the culture supernatant.
Fig. 3 shows the neurite extension effect of the PSEC56 protein, measured by cultivating DRG cells in a media containing the protein.
Fig. 4 shows the neurite extension effect of the PSEC56 protein, measured by cultivating DRG cells in a media containing the protein.
Fig. 5 is a photograph confirming the existence of a fraction containing the Myc-His-PSEC56 protein by Western Blotting with anti-Myc antibodies.

### Best Mode for Carrying out the Invention

Hereinafter, the present invention is specifically illustrated below with reference to Examples, but is not to be construed as being limited thereto.

### [Example 1] Cloning of PSEC56

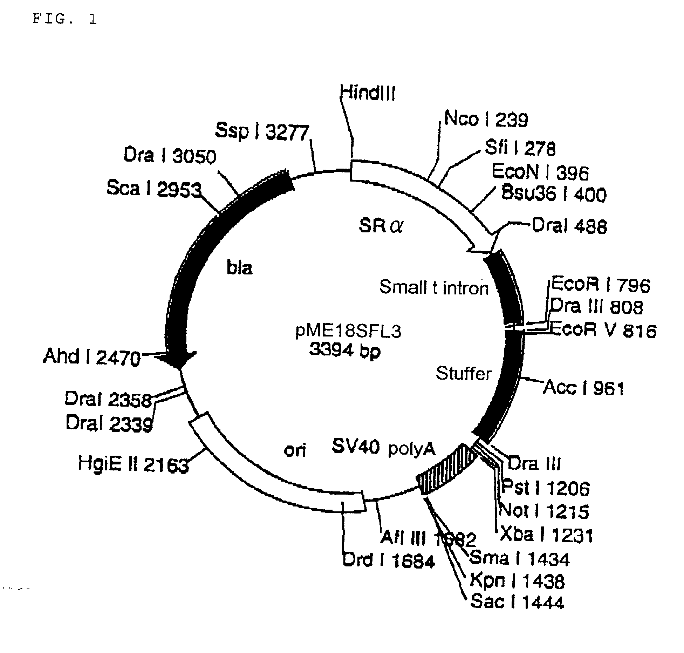

NT-2 nerve progenitor cells (purchased from Stratagene), which are human fetal testis-derived teratocarcinoma cells that can be differentiate into neurons by a treatment with retinoic acid, were used for the cloning of PSEC56. According to the attached instructions, NT-2 cells were cultivated, retinoic acid was added thereto, and the mixture was cultivated for another 2 weeks. These cultivated cells were collected to extract mRNAs according to the literature (J. Sambrook, E. F. Fritsch, and T. Maniatis, Molecular Cloning Second edition, Cold Spring Harbor Laboratory Press, 1989). Then, polyA(+)RNA was purified using oligo dT cellulose. cDNA was cloned from polyA(+)RNA by the oligo-capping method (M. Maruyama and S. Sugano, Gene, 138, 171-174 (1994)). Using oligo-cap linker (SEQ ID NO: 4) and oligo dT primer (SEQ ID NO: 5), BAP (Bacterial Alkaline Phosphatase) treatment, TAP (Tobacco Acid Phosphatase) treatment, RNA ligation, synthesis of primary strand cDNA, and removal of RNA were carried out as described in the literature (Suzuki and Sugano, Protein, Nucleic Acid, and Enzyme, 41, 197-201 (1996), Y. Suzuki et al., Gene, 200, 149-156 (1997)). Next, using 5'- (SEQ ID NO: 6) and 3'- (SEQ ID NO: 7) PCR primers, the primary strand cDNA was converted to double stranded cDNA by PCR (polymerase chain reaction) , and then were digested with SfiI. Then, the cDNA was cloned, with a determined direction, into pME18SFL3 vector (GenBank AB009864, Expression vector, 3392 bp) digested with DraIII (Fig. 1). After a sequencing reaction of the obtained DNA using a DNA sequencing reagent (Dye Terminator Cycle Sequencing FS Ready Reaction Kit, PE Applied Biosystems) following the instructions, the nucleotide sequence of the DNA was analyzed using a DNA sequencer (ABI PRISM 377, PE Applied Biosystems). The longest possible nucleotide sequence from the 5' end of each clone was determined by one pass sequencing. Then, cDNA clone PSEC56 was selected as a clone having a signal sequence by PSORT (Proc. Fourth Int. Conf. Intell. Sys. Mol. Biol., p. 109-115 (1996)) among clones indicating a score of 0.7 or higher according to the translation initiation ATG prediction software for cDNAs (ATGpr developed at Helix Research Institute (A. A. Salamov et al., Bioinformatics, 14, 384-390 (1998)). The full length nucleotide sequence of the cDNA clone was determined, and the amino acid sequence of the region encoding a protein (ORF) was predicted.

The nucleotide sequence of PSEC56, the amino acid sequence of the ORF predicted from the nucleotide sequence of PSEC56, and the predicted signal sequence of PSEC56 are shown in SEQ ID NOs: 1, 2, and 3, respectively.

### [Example 2] Preparation of cells

Preparation of dorsal root ganglion (DRG) cells was conducted according to the method of S. Kim ("No Shinkei Kenkyu no Purotocol: Baiyo kara Kino Kaiseki e (Protocols for Brain and Nerve Research, From Cell Cultivation to Functional Analysis)", K. Mikoshiba, T. Shimizu, edition, Yodosha). More specifically, fetuses were extracted from a female rat (Crj: CD(SD)IGS)at day 18 of pregnancy, and DRGs were removed under a stereoscopic microscope. Cells were dispersed by treating the DRG with 0.25% trypsin. Upon removal of adherent cells other than neurocytes, the neurocytes were plated on a polylysine-coated 96-well plate (5,000 cells/well), and was cultivated for two days in D-MEM containing a test sample and 10% FCS in a CO₂ incubator. As a positive control, nerve growth factor (NGF) was used.

### [Example 3] Measurement of the neurite extension effect

Neurite extension of cells prepared by the above-mentioned method were measured by Cell-based ELISA using the expression of neurofilaments as an indicator of neurite extension. Specifically, cells were immobilized with formalin, were made highly permeable with a surfactant (Triton X-100), and were reacted with specific antibodies (SMI31; Sternberger) against neurofilaments. Next, the aforementioned antibodies were detected with peroxidase-labeled antibodies and peroxidase staining kit T (Sumitomo Bakelite) to measure the absorbance at 450 nm with a microplate reader.

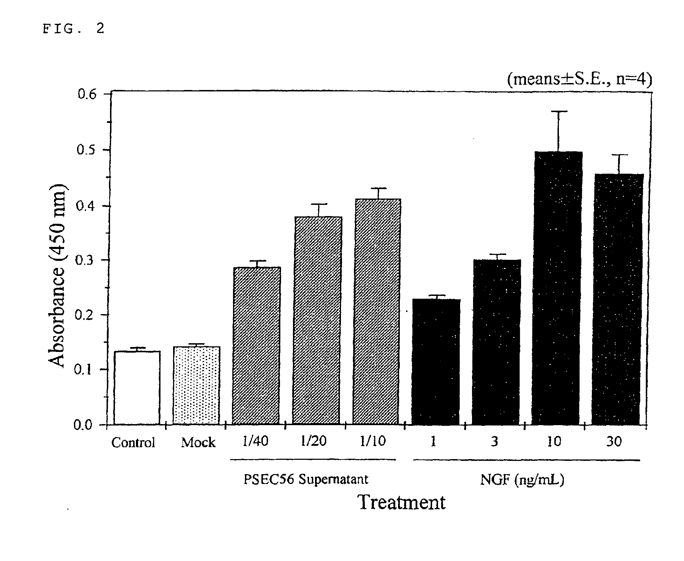

Similarly, to NGF, culture supernatant of COS-7 cells, which were transfected with the PSEC56 gene, as a test substance was revealed to have a neurite extension effect on DRG cells (Fig. 2).

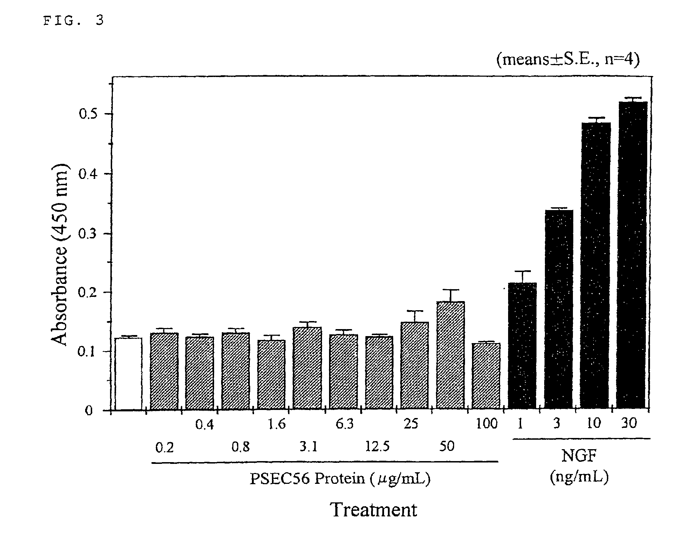

No neurite extension effect on DRG cells at all could be detected for the PSEC56 protein, which were purified from a culture supernatant with a metal affinity column, as a test substance (Fig. 3).

These results mentioned above indicate that not the PSEC56 protein itself has the effect to extend processes of neurocytes, but the main substance causing this effect exists as a molecule other than PSEC56 that is induced and synthesized by the transfection of the PSEC56 cDNA into cells.

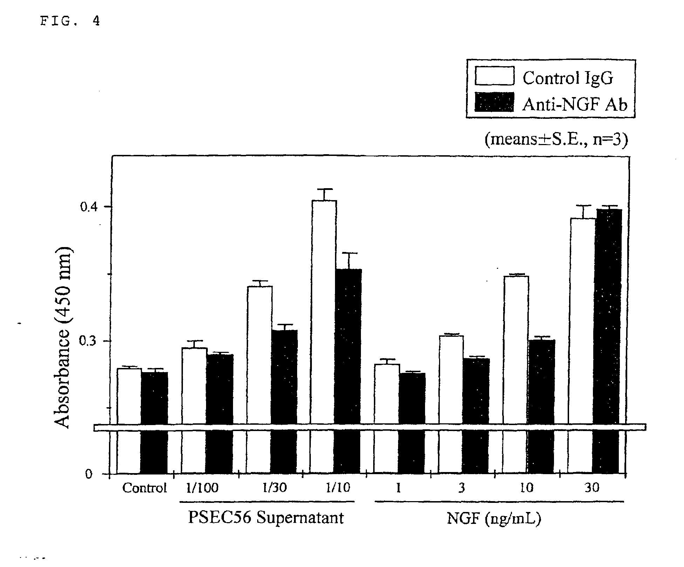

When a culture supernatant of PSEC56 gene-transfected COS-7 cells and the same culture supernatant treated with antibodies (Sigma) against NGF were used as the test substances, it was shown that the neurite extension effect on DRG cells was suppressed by the anti-NGF antibody. A similar effect was detected by other antibodies against NGF (Fig. 4).

### [Example 4] Measurement of nerve growth factors (NGF)

NGF in the culture supernatant were detected by Enzyme-Linked ImmunoSorbent Assay (ELISA) according to the instructions attached to NGF measurement kit from Promega (NGF Eₘₐₓ™ ImmunoAssay System) . More specifically, a 96-well micro test plate was coated with anti-NGF polyclonal antibodies. After blocking, test substances were added thereto, and antigen-antibody reaction was carried out. Following the antigen-antibody reaction by the addition of anti-NGF monoclonal antibodies, anti-rat IgG antibodies labeled with enzymes were added as secondary antibodies. Finally, a substrate, which forms colors through an enzyme reaction, was added thereto. NGF was quantified by measuring the absorbance of the color forming substrate.

When the culture supernatant of PSEC56 gene-transfected COS-7 cells was used as a test substance, NGF was detected from the culture supernatant (approximately 100 ng/mL). However, no NGF could be detected by the Mock transfection.

The results of this Example and Fig. 4 showed that the main molecule having the effect of neurite extension is NGF.

### [Example 5] Purification of PSEC56 protein

The DNA of PSEC56 was inserted into a vector for the addition of Myc and polyhistidine tag (pcDNA3.1(-)/MycHis-A; INVITROGEN). The expression vector was introduced into COS-7 cells by the calcium phosphate method or by the lipofectamine method. 3 days later, the culture supernatant was collected.

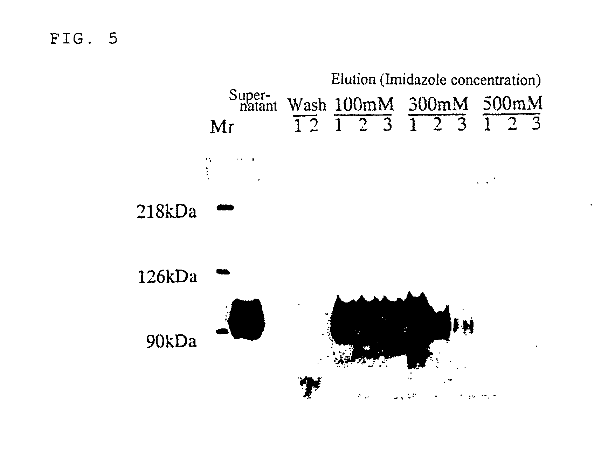

The polyhistidine-attached PSEC56 protein was purified from this culture supernatant using a metal affinity column (HisTrap; Amersham Pharmacia). Specifically, the culture supernatant was passed through a HisTrap column to which nickel ion was added, and the Myc-His-PSEC56 protein bound to the column was eluted using a desired concentration of imidazole solution. Fractions containing the Myc-His-PSEC56 protein were confirmed by Western Blotting using SDS-PAGE and anti-Myc antibody (Fig. 5). Since imidazole was used for the elution from the column, after collecting the fractions of interest, they were desalted by gel filtration using a PD-10 column (Amersham Pharmacia) for later experiments.

### Industrial Applicability

The present invention provides neurotrophic factor expression-inducing agents, a method for inducing the expression of neurotrophic factors, and a method of screening for compounds that regulate the induction of neurotrophic factor expression utilizing PSEC56. This invention enables elucidation of the mechanism leading to the induction and secretion of neurotrophic factors, such as NGF, by PSEC56; and development of novel therapeutic drugs and preventive drugs for diseases of the central nervous system, such as dementia and cerebral infarction, as well as spinal injury and diseases of the peripheral nerves.

## Claims

1. A neurotrophic factor expression-inducing agent, wherein PSEC56 protein or DNA encoding said protein serves as an active ingredient.

2. The agent of claim 1, wherein the neurotrophic factor is NGF.

3. A method for inducing the expression of neurotrophic factors, which comprises the step of expressing PSEC56 protein.

4. The method of claim 3, wherein the neurotrophic factor is NGF.

5. A method of screening for compounds that regulate the induction of neurotrophic factor expression by PSEC56 protein, which comprises the steps of:
(a) contacting a test sample with a transfected cell including an expressive DNA encoding the PSEC56 protein;
(b) detecting the expression of the neurotrophic factor in said cell; and
(c) selecting the compound that enhances or suppresses the induction of neurotrophic factor expression compared to the expression detected in the absence of the test sample (control).

6. The method of claim 5, wherein the neurotrophic factor is NGF.


SEQUENCE LISTING

<110> HELIX RESEARCH INSTITUTE
      WELFIDE CORPORATION

<120> NEUROTROPHIC FACTOR EXPRESSION-INDUCING AGENT

<130> H1-A0002P

<140>
<141>

<150> JP 2000-101300
<151> 2000-03-31

<160> 7

<210> 1
<211> 2641
<212> DNA
<213> Homo sapiens

<220>
<221> CDS
<222> (87)..(1832)

<400> 1
   gagccagttc tgggaggcgg ggggaaggag gttggtggcg actccctcgc tcgccctcac 60
   tgccggcggt cccaactcca ggcacc atg ttc ccc gcg ggc ccc ccc agc cac 113
   Met Phe Pro Ala Gly Pro Pro Ser His
   1 5
   agc ctc ctc cgg ctc ccc ctg ctg cag ttg ctg cta ctg gtg gtg cag 161
   Ser Leu Leu Arg Leu Pro Leu Leu Gln Leu Leu Leu Leu Val Val Gln
   10 15 20 25
   gcc gtg ggg agg ggg ctg ggc cgc gcc agc ccg gcc ggg ggc ccc ctg 209
   Ala Val Gly Arg Gly Leu Gly Arg Ala Ser Pro Ala Gly Gly Pro Leu
   30 35 40
   gaa gat gtg gtc atc gag agg tac cac atc ccc agg gcc tgt ccc cgg 257
   Glu Asp Val Val Ile Glu Arg Tyr His Ile Pro Arg Ala Cys Pro Arg
   45 50 55
   gaa gtg cag atg ggg gat ttt gtg cgc tac cac tac aac ggc act ttt 305
   Glu Val Gln Met Gly Asp Phe Val Arg Tyr His Tyr Asn Gly Thr Phe
   60 65 70
   gaa gat ggc aag aag ttt gat tca agc tat gat cgc aac acc ttg gtg 353
   Glu Asp Gly Lys Lys Phe Asp Ser Ser Tyr Asp Arg Asn Thr Leu Val
   75 80 85
   gcc atc gtg gtg ggt gtg ggg cgc ctc atc act ggc atg gac cga ggc 401
   Ala Ile Val Val Gly Val Gly Arg Leu Ile Thr Gly Met Asp Arg Gly
   90 95 100 105
   ctc atg ggc atg tgt gtc aac gag cgg cga cgc ctc att gtg cct ccc 449
   Leu Met Gly Met Cys Val Asn Glu Arg Arg Arg Leu Ile Val Pro Pro
   110 115 120
   cac ctg ggc tat ggg agc atc ggc ctg gcg ggg ctc att cca ccg gat 497
   His Leu Gly Tyr Gly Ser Ile Gly Leu Ala Gly Leu Ile Pro Pro Asp
   125 130 135
   gcc acc ctc tac ttc gat gtg gtt ctg ctg gat gtg tgg aac aag gaa 545
   Ala Thr Leu Tyr Phe Asp Val Val Leu Leu Asp Val Trp Asn Lys Glu
   140 145 150
   gac acc gtg cag gtg agc aca ttg ctg cgc ccg ccc cac tgc ccc cgc 593
   Asp Thr Val Gln Val Ser Thr Leu Leu Arg Pro Pro His Cys Pro Arg
   155 160 165
   atg gtc cag gac ggc gac ttt gtc cgc tac cac tac aat ggc acc ctg 641
   Met Val Gln Asp Gly Asp Phe Val Arg Tyr His Tyr Asn Gly Thr Leu
   170 175 180 185
   ctg gac ggc acc ttc ttc gac acc agc tac agt aag ggc ggc act tat 689
   Leu Asp Gly Thr Phe Phe Asp Thr Ser Tyr Ser Lys Gly Gly Thr Tyr
   190 195 200
   gac acc tac gtc ggc tct ggt tgg ctg atc aag ggc atg gac cag ggg 737
   Asp Thr Tyr Val Gly Ser Gly Trp Leu Ile Lys Gly Met Asp Gln Gly
   205 210 215
   ctg ctg ggc atg tgt cct gga gag aga agg aag att atc atc cct cca 785
   Leu Leu Gly Met Cys Pro Gly Glu Arg Arg Lys Ile Ile Ile Pro Pro
   220 225 230
   ttc ctg gcc tat ggc gag aaa ggc tat ggg aca gtg atc ccc cca cag 833
   Phe Leu Ala Tyr Gly Glu Lys Gly Tyr Gly Thr Val Ile Pro Pro Gln
   235 240 245
   gcc tcg ctg gtc ttt cac gtc ctc ctg att gac gtg cac aac ccg aag 881
   Ala Ser Leu Val Phe His Val Leu Leu Ile Asp Val His Asn Pro Lys
   250 255 260 265
   gac gct gtc cag cta gag acg ctg gag ctc ccc ccc ggc tgt gtc cgc 929
   Asp Ala Val Gln Leu Glu Thr Leu Glu Leu Pro Pro Gly Cys Val Arg
   270 275 280
   aga gcc ggg gcc ggg gac ttc atg cgc tac cac tac aat ggc tcc ttg 977
   Arg Ala Gly Ala Gly Asp Phe Met Arg Tyr His Tyr Asn Gly Ser Leu
   285 290 295
   atg gac ggc acc ctc ttc gat tcc agc tac tcc cgc aac cac acc tac 1025
   Met Asp Gly Thr Leu Phe Asp Ser Ser Tyr Ser Arg Asn His Thr Tyr
   300 305 310
   aat acc tat atc ggg cag ggt tac atc atc ccc ggg atg gac cag ggg 1073
   Asn Thr Tyr Ile Gly Gln Gly Tyr Ile Ile Pro Gly Met Asp Gln Gly
   315 320 325
   ctg cag ggt gcc tgc atg ggg gaa cgc cgg aga att acc atc ccc ccg 1121
   Leu Gln Gly Ala Cys Met Gly Glu Arg Arg Arg Ile Thr Ile Pro Pro
   330 335 340 345
   cac ctc gcc tat ggg gag aat gga act gga gac aag atc cct ggc tct 1169
   His Leu Ala Tyr Gly Glu Asn Gly Thr Gly Asp Lys Ile Pro Gly Ser
   350 355 360
   gcc gtg cta atc ttc aac gtc cat gtc att gac ttc cac aac cct gcg 1217
   Ala Val Leu Ile Phe Asn Val His Val Ile Asp Phe His Asn Pro Ala
   365 370 375
   gat gtg gtg gaa atc agg aca ctg tcc cgg cca tct gag acc tgc aat 1265
   Asp Val Val Glu Ile Arg Thr Leu Ser Arg Pro Ser Glu Thr Cys Asn
   380 385 390
   gag acc acc aag ctt ggg gac ttt gtt cga tac cat tac aac tgt tct 1313
   Glu Thr Thr Lys Leu Gly Asp Phe Val Arg Tyr His Tyr Asn Cys Ser
   395 400 405
   ttg ctg gac ggc acc cag ctg ttc acc tcg cat gac tac ggg gcc ccc 1361
   Leu Leu Asp Gly Thr Gln Leu Phe Thr Ser His Asp Tyr Gly Ala Pro
   410 415 420 425
   cag gag gcg act ctc ggg gcc aac aag gtg atc gaa ggc ctg gac acg 1409
   Gln Glu Ala Thr Leu Gly Ala Asn Lys Val Ile Glu Gly Leu Asp Thr
   430 435 440
   ggc ctg cag ggc atg tgt gtg gga gag agg cgg cag ctc atc gtg ccc 1457
   Gly Leu Gln Gly Met Cys Val Gly Glu Arg Arg Gln Leu Ile Val Pro
   445 450 455
   ccg cac ctg gcc cac ggg gag agt gga gcc cgg gga gtc cca ggc agt 1505
   Pro His Leu Ala His Gly Glu Ser Gly Ala Arg Gly Val Pro Gly Ser
   460 465 470
   gct gtg ctg ctg ttt gag gtg gag ctg gtg tcc cgg gag gat ggg ctg 1553
   Ala Val Leu Leu Phe Glu Val Glu Leu Val Ser Arg Glu Asp Gly Leu
   475 480 485
   ccc aca ggc tac ctg ttt gtg tgg cac aag gac cct cct gcc aac ctg 1601
   Pro Thr Gly Tyr Leu Phe Val Trp His Lys Asp Pro Pro Ala Asn Leu
   490 495 500 505
   ttt gaa gac atg gac ctc aac aag gat ggc gag gtc cct ccg gag gag 1649
   Phe Glu Asp Met Asp Leu Asn Lys Asp Gly Glu Val Pro Pro Glu Glu
   510 515 520
   ttc tcc acc ttc atc aag gct caa gtg agt gag ggc aaa gga cgc ctc 1697
   Phe Ser Thr Phe Ile Lys Ala Gln Val Ser Glu Gly Lys Gly Arg Leu
   525 530 535
   atg cct ggg cag gac cct gag aaa acc ata gga gac atg ttc cag aac 1745
   Met Pro Gly Gln Asp Pro Glu Lys Thr Ile Gly Asp Met Phe Gln Asn
   540 545 550
   cag gac cgc aac cag gac ggc aag atc aca gtc gac gag ctc aag ctg 1793
   Gln Asp Arg Asn Gln Asp Gly Lys Ile Thr Val Asp Glu Leu Lys Leu
   555 560 565
   aag tca gat gag gac gag gag cgg gtc cac gag gag ctc tgaggggcag 1842
   Lys Ser Asp Glu Asp Glu Glu Arg Val His Glu Glu Leu
   570 575 580
   ggagcctggc caggcctgag acacagaggc ccactgcgag ggggacagtg gcggtgggac 1902
   tgacctgctg acagtcaccc tccctctgct gggatgaggt ccaggagcca actaaaacaa 1962
   tggcagagga gacatctctg gtgttcccac caccctagat gaaaatccac agcacagacc 2022
   tctaccgtgt ttctcttcca tccctaaacc acttccttaa aatgtttgga tttgcaaagc 2082
   caatttgggg cctgtggagc ctggggttgg atagggccat ggctggtccc ccaccatacc 2142
   tcccctccac atcactgaca cagctgagct tgttatccat ctccccaaac tttctctttc 2202
   tttgtacttc ttgtcatccc cactcccagc ccctattcct ctatgtgaca gctggctagg 2262
   acccctctgc cttcctcccc aatcctgact ggctcctagg gaaggggaag gctcctggag 2322
   ggcagcccta cctctcccat gccctttgcc ctcctccctc gcctccagtg gaggctgagc 2382
   tgaccctggg ctgctggagg ccagactggg ctgtagttag cttttcatcc ctaaagaagg 2442
   ctttccctaa ggaaccatag aagagaggaa gaaaacaaag ggcatgtgtg agggaagctg 2502
   catgggtggg tgttagggct atgaaatctt ggatttgggg ctgaggggtg ggagggaggg 2562
   cagagctctg cacactcaaa ggctaaactg gtgtcagtcc tttttccttt tgttccaaat 2622
   aaaagattaa accaatggc 2641

<210> 2

<211> 582

<212> PRT

<213> Homo sapiens

<400> 2

Met Phe Pro Ala Gly Pro Pro Ser His Ser Leu Leu Arg Leu Pro Leu
1               5                   10                  15

Leu Gln Leu Leu Leu Leu Val Val Gln Ala Val Gly Arg Gly Leu Gly
            20                  25                  30

Arg Ala Ser Pro Ala Gly Gly Pro Leu Glu Asp Val Val Ile Glu Arg
        35                  40                  45

Tyr His Ile Pro Arg Ala Cys Pro Arg Glu Val Gln Met Gly Asp Phe
    50                  55                  60

Val Arg Tyr His Tyr Asn Gly Thr Phe Glu Asp Gly Lys Lys Phe Asp
65                  70                  75                  80

Ser Ser Tyr Asp Arg Asn Thr Leu Val Ala Ile Val Val Gly Val Gly
                85                  90                  95

Arg Leu Ile Thr Gly Met Asp Arg Gly Leu Met Gly Met Cys Val Asn
            100                 105                 110

Glu Arg Arg Arg Leu Ile Val Pro Pro His Leu Gly Tyr Gly Ser Ile

        115                 120                 125

Gly Leu Ala Gly Leu Ile Pro Pro Asp Ala Thr Leu Tyr Phe Asp Val

    130                 135                 140

Val Leu Leu Asp Val Trp Asn Lys Glu Asp Thr Val Gln Val Ser Thr

145                 150                 155                 160

Leu Leu Arg Pro Pro His Cys Pro Arg Met Val Gln Asp Gly Asp Phe

                165                 170                 175

Val Arg Tyr His Tyr Asn Gly Thr Leu Leu Asp Gly Thr Phe Phe Asp

            180                 185                 190

Thr Ser Tyr Ser Lys Gly Gly Thr Tyr Asp Thr Tyr Val Gly Ser Gly

        195                 200                 205

Trp Leu Ile Lys Gly Met Asp Gln Gly Leu Leu Gly Met Cys Pro Gly

    210                 215                 220

Glu Arg Arg Lys Ile Ile Ile Pro Pro Phe Leu Ala Tyr Gly Glu Lys

225                 230                 235                 240

Gly Tyr Gly Thr Val Ile Pro Pro Gln Ala Ser Leu Val Phe His Val

                245                 250                 255

Leu Leu Ile Asp Val His Asn Pro Lys Asp Ala Val Gln Leu Glu Thr
            260                 265                 270
Leu Glu Leu Pro Pro Gly Cys Val Arg Arg Ala Gly Ala Gly Asp Phe
        275                 280                 285
Met Arg Tyr His Tyr Asn Gly Ser Leu Met Asp Gly Thr Leu Phe Asp
    290                 295                 300
Ser Ser Tyr Ser Arg Asn His Thr Tyr Asn Thr Tyr Ile Gly Gln Gly
305                 310                 315                 320
Tyr Ile Ile Pro Gly Met Asp Gln Gly Leu Gln Gly Ala Cys Met Gly
                325                 330                 335
Glu Arg Arg Arg Ile Thr Ile Pro Pro His Leu Ala Tyr Gly Glu Asn
            340                 345                 350
Gly Thr Gly Asp Lys Ile Pro Gly Ser Ala Val Leu Ile Phe Asn Val
        355                 360                 365
His Val Ile Asp Phe His Asn Pro Ala Asp Val Val Glu Ile Arg Thr
    370                 375                 380
Leu Ser Arg Pro Ser Glu Thr Cys Asn Glu Thr Thr Lys Leu Gly Asp
385                 390                 395                 400

Phe Val Arg Tyr His Tyr Asn Cys Ser Leu Leu Asp Gly Thr Gln Leu
                405                 410                 415

Phe Thr Ser His Asp Tyr Gly Ala Pro Gln Glu Ala Thr Leu Gly Ala
            420                 425                 430

Asn Lys Val Ile Glu Gly Leu Asp Thr Gly Leu Gln Gly Met Cys Val
        435                 440                 445

Gly Glu Arg Arg Gln Leu Ile Val Pro Pro His Leu Ala His Gly Glu
    450                 455                 460

Ser Gly Ala Arg Gly Val Pro Gly Ser Ala Val Leu Leu Phe Glu Val
465                 470                 475                 480

Glu Leu Val Ser Arg Glu Asp Gly Leu Pro Thr Gly Tyr Leu Phe Val
                485                 490                 495

Trp His Lys Asp Pro Pro Ala Asn Leu Phe Glu Asp Met Asp Leu Asn
            500                 505                 510

Lys Asp Gly Glu Val Pro Pro Glu Glu Phe Ser Thr Phe Ile Lys Ala
        515                 520                 525

Gln Val Ser Glu Gly Lys Gly Arg Leu Met Pro Gly Gln Asp Pro Glu

    530                 535                 540

Lys Thr Ile Gly Asp Met Phe Gln Asn Gln Asp Arg Asn Gln Asp Gly

545                 550                 555                 560

Lys Ile Thr Val Asp Glu Leu Lys Leu Lys Ser Asp Glu Asp Glu Glu

                565                 570                 575

Arg Val His Glu Glu Leu

            580

<210> 3

<211> 26

<212> PRT

<213> Homo sapiens

<400> 3

Met Phe Pro Ala Gly Pro Pro Ser His Ser Leu Leu Arg Leu Pro Leu

1               5                   10                  15

Leu Gln Leu Leu Leu Leu Val Val Gln Ala

            20                  25

<210> 4

<211> 30

<212> RNA

<213> Artificial Sequence

<220>

<223> Description of Artificial Sequence: an

artificially synthesized oligo-cap linker

sequence

<400> 4

agcaucgagu cggccuuguu ggccuacugg 30

<210> 5

<211> 42

<212> DNA

<213> Artificial Sequence

<220>

<223> Description of Artificial Sequence: an

artificially synthesized oligo(dT) adapter primer

sequence

<400> 5

gcggctgaag acggcctatg tggccttttt tttttttttt tt 42

<210> 6

<211> 21

<212> DNA

<213> Artificial Sequence

<220>

<223> Description of Artificial Sequence: an

artificially synthesized primer sequence

<400> 6

agcatcgagt cggccttgtt g 21

<210> 7

<211> 21

<212> DNA

<213> Artificial Sequence

<220>

<223> Description of Artificial Sequence: an

artificially synthesized primer sequence

<400> 7

gcggctgaag acggcctatg t 21